# EUROPEAN PATENT APPLICATION

(11) **EP 3 858 607 A1**
(43) Date of publication of application: **04.08.2021**
(21) Application number: 20864322.1
(22) Date of filing: 16.03.2020
(51) Int. Cl.: B32B 27/02, B32B 27/36, B32B 27/18, B32B 27/40, B32B 27/12, B32B 27/08, B32B 7/12, B32B 33/00, B29C 65/52, A61L 31/12

(54) **MEDICAL COMPOSITE MATERIAL AND MANUFACTURING PROCESS THEREFOR**

(30) Priority: 18.12.2019 CN 201911305690
(71) Applicant: Shandong Juntai Medical Protective Equipment Technology Co., Ltd, Zhangqiu District Jinan Shandong 250200 (CN)
(72) Inventor: The designation of the inventor has not yet been filed
(74) Representative: Sun, Yiming
(86) International application number: PCT/CN2020/079456
(87) International publication number: WO 2021/120426

(57) **Abstract**

The present inventionrelates to amedical composite material and a preparing process thereof. The medical composite material consists of a polyethylene terephthalate fiber, a carbon ion filament fiber, a polyurethane film, and an excipient. The preparing process includes the following steps: preparing a polyester filament fiber; preparinga conductive wire fiber; weaving the polyester filament fiber and the conductive wire fiber into a textile material with an anti-static function; preparing anantimicrobial thin film; and preparing the medical composite material. The medical composite material prepared by the present invention has high waterproof, anti-osmosis and antimicrobial functions, completely wipes out the penetration of the blood, body fluid, flushing fluid of a patient and other pollutants, and realizes a bidirectional protection function; and meanwhile, it has a good moisture transmission function.

## Description

### TECHNICAL FIELD

The present invention relates to a medical composite material and preparing process thereof, and in particular to a medical composite material with waterproof, anti-osmosis, anti-pollution, anti-moisture permeation, anti-static, hypochlorite rinsing resistant and deflocculation functions and a preparing process thereof, and belongs to the technical field of medical materials.

### BACKGROUND

In the prior art, the commonly used materials of medical fabrics are cotton and disposable non-woven materials.

The cotton material: it has no basic barrier function, is easily permeable to blood and body fluid of a patient and other pollutants, leads to cross-infectiontomedical staff and patients; produces a large amount of floccule, which will cause wound infection and large-areainfection in medical institutions; it has no antimicrobial function, and cannot give the best safety protection to the medical staff and patients; and cotton operating-room medical fabric has been eliminated by developed countries in Europe and America.

The disposable non-woven material: although it has a certain barrier function, it cannot achieve complete protection when a large amount of pressurized blood, body fluids and flushing fluids are encountered; it has a moisture transmission ratethatis too low and sultry, which will increase the workload of surgical medical staff; it has a high cost for single-time use; it produces a large amount of medical waste, and incineration of it will produce dioxin and the like harmful substances and pollute the environment.

In view of the above problems, the inventor develops a novel medical composite material, which solves the existing problems of the cotton material and the disposable non-woven fabric; and provides safer protective materials for the medical staff and patients.

### SUMMARY

The present invention provides a medical composite material and a preparing process thereof, and in particular relates to a medical composite material with waterproof, anti-osmosis, anti-moisture permeation, anti-static, hypochlorite rinsing resistant, antimicrobial and deflocculation functions.

The technical solutions of the present invention are as follows.

Provided is a medical composite material, including the following components in parts by weight:
55-65 parts of a polyethylene terephthalate fiber, 8-12 parts of a carbon ion filament fiber, 18-23 parts of a polyurethane film, and 5-15 parts of an excipient.

Preferably, the medical composite materialincludes 60 parts by weight of the polyethylene terephthalate fiber, 10 parts by weight of the carbon ion filament fiber, 20 parts by weight of the polyurethane film, and 10 parts by weight of the excipient; the excipient is a polyurethane adhesive.

Provided is a process for preparing the medical composite material of the present invention, specifically including the steps of:
(1) preparing a polyester filament fiber, making preliminary improvement on the polyester filament fiber, and using an air deformation process to reduce the hardness and smoothness degrees of the texture of the surface of the polyester filament fiber, wherein the polyester filament fiber is the polyethylene terephthalate (PET) fiber;
(2) preparing a conductive wire fiber
   using a terylene filament fiber with a circular fiber section, fully permeating carbon ions into the terylene filament fiber; then attaching and fixing the carbon ions onto the surface of the fiber by employing a coating process, thereby yield a conductive wire fiber with a thickness of about 1 micrometer;
(3) weaving the polyester filament fiber obtained in the step (1) and the conductive wire fiber obtained in the step (2) into an antistatic textile material, wherein a distance among the conductive wires is 1 cm;
(4) preparing an antimicrobial thin film
   utilizing polyurethane (PU) to prepare a thin film having a thickness of 0.012-0.035 mm as the antimicrobial film,
   wherein the antimicrobial film has a structure of 13.95 billion micropores per square centimeter, each micropore is 280 nm in diameter; in the present invention, the diameter of the micropore is 2,000 times smaller than the diameter of a liquid water molecular cluster that is 0.1 mm - 7 mm and 70 times larger than the diameter of a vapor water molecular cluster that is 3 nm-4 nm; and
(5) preparing the medical composite material: disposing the antibacterial film as an intermediate layer between the polyester filament fiber as an upper layer and the conductive wire fiber as a lower layer, and compounding the three layers of materials by a roller-coating and compounding process employing a polyester adhesive, to yield the medical composite material.

The present invention further includes printing and dyeing the medical composite material such that the printed and dyed medical composite material meets the requirements of grade 4 standard (GB/T7069-1997). The step of printing and dyeing specifically includes:
(1) printing and dyeing of a sample
   using a material of 40 cm x100 cm as a sample; immersing the sample in a printing and dying additive in a glass or enamel container and allowing the sample to stand, and then immersing the sample in a hypochlorite solution for 60 min, wherein an effective chlorine content in the hypochlorite solution is 2 g/L, and a pH of the hypochlorite solution is 11.0 ± 0.2; and
(2) printing and dyeing of a material
   examining the sample, and if it is qualified, printing and dyeing the medical composite material in batches.

Compared with the prior art, the present invention has the following advantages:
The medical composite materialprepared by the present inventionhas high waterproof, anti-osmosis and antimicrobial functions, completely wipes out the penetration of the blood, body fluid, flushing fluid of a patient and other pollutants, and realizes a bidirectional protection function; and meanwhile, it has a good moisture transmission function. It increases the comfort of the medical staff and patients and is conducive to the use of surgical clothing, surgical sheet and instrument sheet. The antistatic function of itavoidsthe operation risk caused by static electricity. No floccule is produced by it, which avoids the infection risk caused by the floccule. The medical composite material is resistant to hypochlorite rinsing, increases washing times and reduces single use cost. The present invention provides a reusable environment-friendly material, and in processing process of it, no dioxin and other carcinogens is produced. In addition to the medical field, the medical composite materialprepared by the present invention can also be used for the aged, infant, and the like civilian fields, and partial of military fields.

### DETAILED DESCRIPTION

The present invention will be further described in connection with specific examples below. The advantages and features of the present invention will become more apparent with the description. However, the examples are only exemplary, and do not limit the scope of the present invention in any way. It should be understood by those skilled in the art that modifications and substitutions can be made in the details and forms of the technical solutions of the present invention without departing from the spirit and scope of the present invention, but all of these modifications and substitutions fall within the claimed scope of the present invention.

### Example 1: a medical composite material and a preparing process thereof

Provided is a medical composite materialincluding the following components in parts by weight:
60 parts of the polyethylene terephthalate fiber, 10 parts of the carbon ion filament fiber, 20 parts of the polyurethane film, and 10 parts of the excipient. The excipient is a polyurethane adhesive.

Provided is a process for preparing the afore-mentionedmedical composite material, specifically including the steps of:
(1) preparing a polyester filament fiber, making preliminary improvement on the polyester filament fiber, and using an air deformation process to reduce the hardness and smoothness degrees of the texture of the surface of the polyester filament fiber, wherein the polyester filament fiber is the polyethylene terephthalate (PET) fiber;
(2) preparing a conductive wire fiber
   using a terylene filament fiber with a circular fiber section, fully permeating carbon ions into the terylene filament fiber; then attaching and fixing the carbon ions onto the surface of the fiber by employing a coating process, thereby yield a conductive wire fiber with a thickness of about 1 micrometer;
(3) weaving the polyester filament fiber obtained in the step (1) and the conductive wire fiber obtained in the step (2) into an antistatic textile material, wherein a distance among the conductive wires is 1 cm;
(4) preparing an antimicrobial thin film
   utilizing polyurethane (PU) to prepare a thin film having a thickness of 0.015-0.025 mm as the antimicrobial film,
   wherein the antimicrobial film has a structure of 13.95 billion micropores per square centimeter, each micropore is 280 nm in diameter; and
(5) preparing the medical composite material: disposing the antibacterial film as an intermediate layer between the polyester filament fiber as an upper layer and the conductive wire fiber as a lower layer, and compounding the three layers of materials by a roller-coating and compounding process employing a polyester adhesive, to yield the medical composite material.

### Example 2: a medical composite material and a preparing process thereof

Provided is a medical composite materialincluding the following components in parts by weight:
63 parts of the polyethylene terephthalate fiber, 12 parts of the carbon ion filament fiber, 22 parts of the polyurethane film, and 13 parts of the excipient. The excipient is a polyurethane adhesive.

Provided is a process for preparing the afore-mentioned medical composite material, specifically including the steps of:
(1) preparing a polyester filament fiber, making preliminary improvement on the polyester filament fiber, and using an air deformation process to reduce the hardness and smoothness degrees of the texture of the surface of the polyester filament fiber, wherein the polyester filament fiber is the polyethylene terephthalate (PET) fiber;
(2) preparing a conductive wire fiber
   using a terylene filament fiber with a circular fiber section, fully permeating carbon ions into the terylene filament fiber; then attaching and fixing the carbon ions onto the surface of the fiber by employing a coating process, thereby yield a conductive wire fiber with a thickness of about 1 micrometer;
(3) weaving the polyester filament fiber obtained in the step (1) and the conductive wire fiber obtained in the step (2) into an antistatic textile material, wherein a distance among the conductive wires is 1 cm;
(4) preparing an antimicrobial thin film
   utilizing polyurethane (PU) to prepare a thin film having a thickness of 0.012-0.035 mm as the antimicrobial film,
   wherein the antimicrobial film has a structure of 13.95 billion micropores per square centimeter, each micropore is 280 nm in diameter; and
(5) preparing the medical composite material: disposing the antibacterial film as an intermediate layer between the polyester filament fiber as an upper layer and the conductive wire fiber as a lower layer, and compounding the three layers of materials by a roller-coating and compounding process employing a polyester adhesive, to yield the medical composite material.

### Example 3: a medical composite material and a preparing process thereof

Provided is a medical composite materialincluding the following components in parts by weight:
56 parts of the polyethylene terephthalate fiber, 9 parts of the carbon ion filament fiber, 18 parts of the polyurethane film, and 8 parts of the excipient. The excipient is a polyurethane adhesive.

Provided is a process for preparing the afore-mentioned medical composite material, specifically including the steps of:
(1) preparing a polyester filament fiber, making preliminary improvement on the polyester filament fiber, and using an air deformation process to reduce the hardness and smoothness degrees of the texture of the surface of the polyester filament fiber, wherein the polyester filament fiber is the polyethylene terephthalate (PET) fiber;
(2) preparing a conductive wire fiber
   using a terylene filament fiber with a circular fiber section, fully permeating carbon ions into the terylene filament fiber; then attaching and fixing the carbon ions onto the surface of the fiber by employing a coating process, thereby yield a conductive wire fiber with a thickness of about 1 micrometer;
(3) weaving the polyester filament fiber obtained in the step (1) and the conductive wire fiber obtained in the step (2) into an antistatic textile material, wherein a distance among the conductive wires is 1 cm;
(4) preparing an antimicrobial thin film
   utilizing polyurethane (PU) to prepare a thin film having a thickness of 0.025-0.035 mm as the antimicrobial film,
   wherein the antimicrobial film has a structure of 13.95 billion micropores per square centimeter, each micropore is 280 nm in diameter; and
(5) preparing the medical composite material: disposing the antibacterial film as an intermediate layer between the polyester filament fiber as an upper layer and the conductive wire fiber as a lower layer, and compounding the three layers of materials by a roller-coating and compounding process employing a polyester adhesive, to yield the medical composite material.

### Example 4

The present invention further includes printing and dyeing the medical composite material such that the printed and dyed medical composite material meets the requirements of grade 4 standard (GB/T7069-1997). The step of printing and dyeing specifically includes:
(1) printing and dyeing of a sample
   using a material of 40 cm × 100 cm as a sample; immersing the sample in a printing and dying additive in a glass or enamel container and allowing the sample to stand, and then immersing the sample in a hypochlorite solution for 60 min, wherein an effective chlorine content in the hypochlorite solution is 2 g/L, and a pH of the hypochlorite solution is 11.0 ± 0.2; and
(2) printing and dyeing of a material
   examining the sample, and if it is qualified, printing and dyeing the medical composite material in batches.

**Test Example** The composite material of the present invention is detected, and the detection results are shown in Table 1:

**Table 1**

| detection items | Actual detection result | Basis of detection |
|---|---|---|
| Charge, unit: uC/piece | 0.56 | GB12014-2009 |
| | | 22.0°C, 34%RH |
| Water resistance (hydrostatic pressure method) (mmH₂O) (the observe side) | >3000 | GB/T 4744-2013 (60cmH₂O/min, water temperature of 20°C) |
| Colorfastness (grade) to hypochlorite bleaching, discoloration | 4 | GB/T 7069-1997 |
| Water vapor permeability (g/(m²·d)) (the obverse side towards the water) | 7.8×10³ | GB/T 12704.2-2009 f Method B (at a temperature of 38°C and a relative humidity of 50%) |
| Moisture permeability (g/m²/24hrs) after washing 20 times, at a temperature of 38°C±2°C and a humidity of 50%±2% | 9774.9 | GB/T 12704.2-2009 Method B; inverted cup evaporation method |
| Hydrostatic pressure after washing 20 times (mm) | 8760 | GB/T 4744-2013; water pressure rise rate: 10 cm H₂O/min |
| Moisture permeability (g/m²/24hrs) after washing 100 times, at a temperature of 38°C±2°C and a humidity of 50%±2% | 10586.0 | GB/T 12704.2-2009 Method B; inverted cup evaporation method |
| Hydrostatic pressure after washing 100 times (mm) | 855 | GB/T 4744-2013; water pressure rise rate: 10 cm H₂O/min |

Furthermore, upon detection, the cotton cloth, which was used as a control sample, was penetrated by *Staphylococcus aureus* under wet conditions. The total number of colonies growing on 5 medium plates was more than 5. Under dry conditions, the total number of colonies penetrated in 10 samples was 187 colony-forming unit (cfu), which was much higher than 15 cfu. The medical composite material of the present invention can effectively prevent the penetration of *Staphylococcus aureus* under wet conditions and black spore variants of *Bacillus subtilis* under dry conditions. Meanwhile, upon detection, the cleanliness of the medical composite material of the present invention - microorganism ≤ 300 cfu/dm²; cleanliness - particulate matters ≤ 3.5 index of particulate matters (IPM); flocculus ≤ 4.0 Iog10; dry and wet bursting strength ≥ 40 KPa; dry and wet fracture strength: ≥ 20 N.

## Claims

1. A medical composite material, comprising the following components in parts by weight:
55-65 parts of a polyethylene terephthalate fiber, 8-12 parts of a carbon ion filament fiber, 18-23 parts of a polyurethane film, and 5-15 parts of an excipient.

2. The medical composite material of claim 1, comprising the following components in parts by weight:
60 parts of the polyethylene terephthalate fiber, 10 parts of the carbon ion filament fiber, 20 parts of the polyurethane film, and 10 parts of the excipient; wherein the excipient is a polyurethane adhesive.

3. A process for preparing the medical composite material of claim 1 and 2, comprising the steps of:
(1) preparing a polyester filament fiber, making preliminary improvement on the polyester filament fiber, and using an air deformation process to reduce the hardness and smoothness degrees of the texture of the surface of the polyester filament fiber, wherein the polyester filament fiber is the polyethylene terephthalate (PET) fiber;
(2) preparing a conductive wire fiber
using a terylene filament fiber with a circular fiber section, fully permeating carbon ions into the terylene filament fiber; then attaching and fixing the carbon ions onto the surface of the fiber by employing a coating process, thereby yield a conductive wire fiber with a thickness of about 1 micrometer;
(3) weaving the polyester filament fiber obtained in the step (1) and the conductive wire fiber obtained in the step (2) into an antistatic textile material, wherein a distance among the conductive wires is 1 cm;
(4) preparing an antimicrobial thin film
utilizing polyurethane (PU) to prepare a thin film having a thickness of 0.012-0.035 mm as the antimicrobial film,
wherein the antimicrobial film has a structure of billion micropores per square centimeter, each micropore is 280 nm in diameter; and
(5) preparing the medical composite material: disposing the antibacterial film as an intermediate layer between the polyester filament fiber as an upper layer and the conductive wire fiber as a lower layer, and compounding the three layers of materials by a roller-coating and compounding process employing a polyester adhesive, to yield the medical composite material.

4. The preparing process of claim 3, further comprising printing and dyeing the medical composite material, wherein the step of printing and dyeing specifically comprises:
(1) printing and dyeing of a sample
using a material of 40 cm x100 cm as a sample; immersing the sample in a printing and dying additive in a glass or enamel container and allowing the sample to stand, and then immersing the sample in a hypochlorite solution for 60 min, wherein an effective chlorine content in the hypochlorite solution is 2 g/L, and a pH of the hypochlorite solution is 11.0 ± 0.2; and
(2) printing and dyeing of a material
examining the sample, and if it is qualified, printing and dyeing the medical composite material in batches.
